(19)

Europäisches
Patentamt

European
Patent Office

Office européen
des brevets

(11) **EP 4 702 849 A1**

(12) **EUROPEAN PATENT APPLICATION**
published in accordance with Art. 153(4) EPC

(43) Date of publication:
**04.03.2026 Bulletin 2026/10**

(21) Application number: **24721168.3**

(22) Date of filing: **19.03.2024**

(51) International Patent Classification (IPC):
**A23L 33/15** $^{(2016.01)}$ **A23L 33/16** $^{(2016.01)}$
**C12Q 1/6883** $^{(2018.01)}$ **G01N 33/50** $^{(2006.01)}$
**G06Q 30/0601** $^{(2023.01)}$ **G16H 10/00** $^{(2018.01)}$
**G16H 20/60** $^{(2018.01)}$

(52) Cooperative Patent Classification (CPC):
**A23L 33/15; A23L 33/16; G06Q 30/0621;
G06Q 30/0631; G06Q 50/04; G16H 20/60;
G16H 50/30;** C12Q 2600/106; C12Q 2600/156

(86) International application number:
**PCT/ES2024/070173**

(87) International publication number:
**WO 2024/223961 (31.10.2024 Gazette 2024/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **28.04.2023 ES 202330341**

(71) Applicant: **Dulcesa, S.L.U.**
**46702 Gandia (Valencia) (ES)**

(72) Inventor: **JUAN FERNÁNDEZ, Rafael**
**46702 Gandia Valencia (ES)**

(74) Representative: **Ungria López, Javier**
**Avda. Ramón y Cajal, 78**
**28043 Madrid (ES)**

(54) **METHOD FOR DESIGNING A CUSTOMISED FOOD FOR AN INDIVIDUAL FOR SUBSEQUENT MANUFACTURE THEREOF**

(57) The invention relates to a method for designing a customised food for an individual for subsequent manufacture thereof, wherein the food is enriched with at least one nutrient selected from calcium, magnesium, zinc, vitamin A, vitamin B6, vitamin B9, vitamin B12, vitamin C, vitamin D, vitamin E and vitamin K, and wherein the method comprises determining the individual's genotype at at least one given SNP position and calculating the individual's daily intake of the aforementioned nutrients. Another object of the invention is a computer-implemented system to carry out said method.

**Description**

**FIELD OF THE INVENTION**

The present invention relates to a method and system for manufacturing a customised food adapted to provide nutritional benefits according to the needs of each individual or user.

**BACKGROUND OF THE INVENTION**

The concept of Nutrigenomics is relatively recent and essentially refers to everything that is related to the expression of genes, its variability between individuals and the influence of food nutrients or compounds on the degree of expression of these genes and therefore on the onset or prevention of diseases. Nutrigenomics is a type of nutrition research that has signs of a promising future for improving health and preventing the development of chronic diseases related to diet and lifestyles. Moreover, a correlation has been observed between monogenic and multifactorial diseases and diet, aiming to descriptively highlight those polymorphisms that have been shown to be directly influenced by nutritional or bioactive compounds. The field of Nutrigenomics is in a clear process of expansion and development, progressively gaining momentum from both the field of research and the point of view of business. In view of the research conducted, it seems that the development of this science will not be easy, although nowadays the various advances in the "omic" sciences have increased the speed of research and have consequently facilitated its business application. Interest is currently focused on recognising genes that could be related to the development of diseases or modified morphological traits due to their mutations (mainly due to the diet).

The development of pharmacogenomics made it possible to subsequently make progress in the study on the interrelationship between genes, diet and illness, leading to the emergence of the recent field of nutritional genomics called nutrigenomics and nutrigenetics. Nutritional genomics is a type of nutrition research that has signs of a promising future along with the rise of new challenges and it aims to improve health and prevent diet-derived alterations in a much more individualised and customised way.

In nutrition, the food pyramid reflects common recommendations for all of society, preventive health ideas that mention serving sizes, food, but always from a population perspective. This concept, however, is evolving and it is now known that the genetic information of each person determines their nutritional and metabolic state. Therefore, in the current century exclusively community nutrition is evolving into the design of customised nutrition, since each individual has different biochemical and metabolic traits due to their genetic characteristics. Therefore, it seems that customised diets and recommendations could be designed based on these genetic characteristics. This high variability in response can significantly alter the effectiveness of the general nutritional recommendations that have traditionally been made when they are applied at the individual level. Current research suggests that despite there being a set of general dietary guidelines for the entire population, said guidelines may not suit everyone's needs. Different genetic variations cause differences in nutritional requirements and different genotypes contribute to a greater or lesser predisposition to suffer certain chronic diseases. The promising contribution of this type of nutrition research envisions new challenges to determine which genes are related in the different nutritional processes. After verifying that genetic differences between individuals cause different reactions to nutrients, the idea of combining genetics and nutrition arises, in which the new field of nutritional genomics is developed.

In this context, it is worth noting that of the 3 billion base pairs of human DNA, 99.9% of DNA sequences are identical; however, these differences between different individuals have a high biological significance and are what determine phenotypic differences. Our genetic code is what makes us who we are, sharing genes and functions even through each individual is unique and different, which is expressed by the minimal existing genetic variations. The most common form of genetic variability are single nucleotide polymorphisms (SNP), which refer to the variation that affects a single nucleotide in the DNA sequence among individuals in a population. Variations of this type must occur in at least 1% of the population to be considered an SNP.

Obesity is a global epidemic in which more than 35% of the world's population (2.1 billion people) are estimated to have problems related to being overweight or obese based on body mass index (BMI). Obesity is associated with a multitude of health problems, including dyslipidaemias, cardiovascular diseases (CVD), type 2 diabetes mellitus (T2DM), non-alcoholic fatty liver disease (NAFLD) and some types of cancer, and these problems entail important economic and social costs for health systems. Systematic analyses have revealed that obesity and being overweight caused 3.4 million deaths in 2010. Long-term consumption of unbalanced diets (high in calories, fats, fructose and a high ratio of omega-6/omega-3 fatty acids), along with leading a sedentary lifestyle, contributes to the development of obesity and its associated complications. Furthermore, it is recognised that genetic and epigenetic interactions with the environment and with factors associated with dietary intake or physical activity play an important role in determining phenotypes. Recent advances in genome sequencing and large cohort studies are clarifying the involvement and interaction of these factors in chronic disorders, including obesity, which open a new field to customise strategies in precision or customised medicine, which relates to therapy for the disease based on interindividual and mainly genetic differences, in addition to environmental or microbiome differences.

Thus, precision nutrition is an important part of precision medicine that can help establish nutritional guidelines for specific subgroups instead of conventional population-based advising. A large number of genetic association studies have identified single nucleotide polymorphism (SNP) genetic markers related to obesity, dyslipidaemia, future treatments, therapeutic targets and dietary and lifestyle recommendations. In this sense, dietary advice programs, mainly those aimed at weight loss, fat consumption, sugars and the treatment of metabolic disorders (i.e., insulin resistance, dyslipidaemias, fatty liver), are of great importance, in addition to the study of nutritional associations based on certain nutrients and bioactive dietary compounds that can modify epigenetic marks and that review gene expressions.

Currently, there are several companies that offer an analysis service in which a sample is taken from the user to determine potential predispositions to different diagnoses (without necessarily having to reach the clinical or pathological limit).

However, there is still a need in this technical field to provide products and services to meet the demands arising from greater consumer awareness of the needs to eat a balanced diet and which take into account the individualised characteristics of the subject.

## DESCRIPTION OF THE INVENTION

In a first aspect, the invention relates to a method for designing a customised food for an individual, giving rise to a formula that defines the nutritional and ingredient composition of said customised food for subsequent manufacture thereof, wherein the customised food is enriched with at least one nutrient selected from calcium, magnesium, zinc, vitamin A, vitamin B6, vitamin B9, vitamin B12, vitamin C, vitamin D, vitamin E and vitamin K, as well as any of the combinations thereof and wherein the method comprises:

(a) determining the individual's genotype at at least one SNP position from among rs17216707 (CYP24A1), rs11144134 (TRPM6), rs1532423 (CA1), rs11645428 (PKD1L2), rs4654748 (NBPF3), rs1801133 (MTHFR), rs601338 (FUT2), rs33972313 (SLC23A1), rs10741657 (CYP2R1), rs12272004 (APOA5) and rs9923231 (VKORC1), as well as any of the combinations thereof, from a DNA sample of said individual;
(b) calculating the individual's daily intake of at least one nutrient selected from calcium, magnesium, zinc, vitamin A, vitamin B6, vitamin B9, vitamin B12, vitamin C, vitamin D, vitamin E and vitamin K, as well as any of the combinations thereof;

where:

- if the genotype for rs17216707 (CYP24A1) is TT or TC, and/or if the daily calcium intake is low (less than 800 mg daily), the customised food is enriched in calcium;
- if the genotype for rs11144134 (TRPM6) is AA or AG, and/or if the daily magnesium intake is low (less than 375 mg daily), the customised food is enriched in magnesium;
- if the genotype for rs1532423 (CA1) is TT or TC, and/or if the daily zinc intake is low (less than 10 mg daily), the customised food is enriched in zinc;
- if the genotype for rs11645428 (PKD1L2) is CC, and/or if the daily vitamin A intake is low (less than 800 µg daily), the customised food is enriched in vitamin A;
- if the genotype for rs4654748 (NBPF3) is CC or CT, and/or if the daily vitamin B6 intake is low (less than 1.4 mg daily), the customised food is enriched in vitamin B6;
- if the genotype for rs1801133 (MTHFR) is TT or TC, and/or if the daily vitamin B9 intake is low (less than 200 µg daily), the customised food is enriched in vitamin B9;
- if the genotype for rs601338 (FUT2) is GG or GA, and/or if the daily vitamin B12 intake is low (less than 2.5 µg daily), the customised food is enriched in vitamin B12;
- if the genotype for rs33972313 (SLC23A1) is AA or AG, and/or if the daily vitamin C intake is low (less than 80 mg daily), the customised food is enriched in vitamin C;
- if the genotype for rs10741657 (CYP2R1) is CC or CT, and/or if the daily vitamin D intake is low (less than 5 µg daily), the customised food is enriched in vitamin D;
- if the genotype for rs12272004 (APOA5) is CC, and/or if the daily vitamin E intake is low (less than 12 mg daily), the customised food is enriched in vitamin E; and/or
- if the genotype for rs9923231 (VKORC1) is AA or AG, and/or if the daily vitamin K intake is low (less than 75 µg daily), the customised food is enriched in vitamin K.

In a particular embodiment of the invention, the food is a beverage.

Particularly, the calculation of the individual's daily intake of at least one nutrient selected from calcium, magnesium, zinc, vitamin A, vitamin B6, vitamin B9, vitamin B12, vitamin C, vitamin D, vitamin E and vitamin K, as well as any of the combinations thereof, can be carried out by determining the individual's consumption of at least one food selected from a group consisting of: dairy (such as, for example, whole milk, semi-skimmed milk, non-fat or skimmed milk, whole yoghurt, non-fat yoghurt, curd, white or fresh cheese, aged, semi-aged or cream cheese, custard, flan, pudding and/or ice cream); eggs, meat or fish (such as, for example, chicken eggs, chicken with skin, skinless chicken, veal, pork, lamb, beef liver, cured meat, sausages and the like, pâtés, foie gras, hamburgers, fried fish, white fish, oily fish, canned fish, clams, mussels, oysters, squid, baby squid, cuttlefish, octopus and/or seafood such as shrimp, crab, prawn or lobster); vegetables, legumes or fruit (such as, for example, spinach or chard, cabbage, cauliflower, broccoli, lettuce, endives, escarole, tomato, onion, carrot, pumpkin, green beans, aubergine, courgette, cucumber, pepper, asparagus, artichoke, corn, legumes such as lentils, chickpeas, pinto or white beans, or fruit such as orange, tangerine, banana, apple, pear, grapes, strawberries, cherries, peach, nectarine, apricot, watermelon, melon, kiwi, olives and/or nuts such as almonds, peanuts, pine nuts, hazelnuts, etc.); bread, cereals and the like (such as, for example, white or wholegrain bread, cooked, roasted or fried potatoes, cooked rice, pasta such as spaghetti, noodles, macaroni and the like and/or pizza); oils, fats and the like (such as, for example, olive, sunflower, corn or soybean oil, margarine added to bread or food, butter added to bread or food, biscuits, pastries such as croissant, doughnut, cupcake, sponge cake, cake or the like and/or chocolate, bonbons and the like); precooked and miscellaneous beverages (such as, for example, red, white or rosé wine, beer, brandy, gin, rum, whisky, vodka, 40° spirits, sugar-sweetened or sugar-free soft drinks, packaged fruit juice, coffee, croquettes, mayonnaise, tomato sauce, salt, garlic, jams, honey, sugar, etc.), wherein each food is registered (for example, in a database) with its nutritional information and, in particular, the amount of at least one nutrient selected from calcium, magnesium, zinc, vitamin A, vitamin B6, vitamin B9, vitamin B12, vitamin C, vitamin D, vitamin E and vitamin K, as well as any of the combinations thereof, that is present in it.

Preferably, the method may additionally include determining user preferences that will be taken into account in the design of the customised food, wherein said user preferences may consist of the base or main ingredient of the food (for example, in the case of a beverage, if it is a dairy beverage and, in that case, the individual's preferred type of milk, for example, animal or plant-based milk (for example, oat milk)), flavour of the food (for example, a beverage with a chocolate flavour, vanilla flavour, coconut flavour, etc.), preference for enriching at least one nutrient, preferably selected from protein and fibre, and/or preference for reducing at least one nutrient that may be cholesterol. Each of these preferences will be associated with an ingredient, which in turn will have established information regarding its nutritional and allergen content. In this regard, the result of the method will be a formula defined on the basis of the nutritional and ingredient composition of said customised food for subsequent manufacture thereof.

In another particular embodiment, the method may comprise an additional step of storing said formula (in particular, the ingredients and nutritional information corresponding to the customised food) in a QR code. The nutritional information will be calculated from the nutritional data of each ingredient that makes up the beverage, wherein said nutritional information may be collected in a database of the system.

In another particular embodiment, the method may comprise an additional step of storing the ingredients and the nutritional information corresponding to the customised food on a label that can be subsequently printed, which may additionally include data on the amount of total food to be manufactured, taking into account 3% by weight of shrinkage that is wasted during the manufacturing process.

Another object of the invention is the method described above, wherein said method is implemented by computer.

In addition, the object of the invention is a computer-implemented system configured to design a customised food, giving rise to a formula that defines the nutritional and ingredient composition of said customised food for subsequent manufacture thereof, wherein said food is enriched with at least one nutrient selected from calcium, magnesium, zinc, vitamin A, vitamin B6, vitamin B9, vitamin B12, vitamin C, vitamin D, vitamin E and vitamin K, as well as any of the combinations thereof, wherein said system comprises a processing unit comprising a memory and wherein said processing unit is configured to execute the following instructions:

a) in the memory, store user data corresponding to the individual's genotype at at least one SNP position from among rs17216707 (CYP24A1), rs11144134 (TRPM6), rs1532423 (CA1), rs11645428 (PKD1L2), rs4654748 (NBPF3), rs1801133 (MTHFR), rs601338 (FUT2), rs33972313 (SLC23A1), rs10741657 (CYP2R1), rs12272004 (APOA5) and rs9923231 (VKORC1);

b) in the memory, store user data corresponding to the daily intake of at least one nutrient selected from calcium, magnesium, zinc, vitamin A, vitamin B6, vitamin B9, vitamin B12, vitamin C, vitamin D, vitamin E and vitamin K, as well as any of the combinations thereof;

c) compare said genotype of the individual with a first reference parameter, wherein said first reference parameter refers to a genotype at an SNP position consisting of TT or TC for rs17216707 (CYP24A1), AA or AG for rs11144134 (TRPM6), TT or TC for rs1532423 (CA1), CC for rs11645428 (PKD1L2), CC or CT for rs4654748 (NBPF3), TT or TC for rs1801133 (MTHFR), GG or GA for rs601338 (FUT2), AA or AG for rs33972313 (SLC23A1), CC or CT for rs10741657 (CYP2R1), CC for rs12272004 (APOA5) and AA or AG for rs9923231 (VKORC1);

d) compare said daily intake of the individual with a second reference parameter, wherein said second reference parameter refers to a recommended daily intake of at least one nutrient consisting of 800 mg of calcium daily, 375 mg of magnesium daily, 10 mg of zinc daily, 800 $\mu$g of vitamin A daily, 1.4 mg of vitamin B6 daily, 200 $\mu$g of vitamin B9 daily, 2.5 $\mu$g of vitamin B12 daily, 80 mg of vitamin C daily, 5 $\mu$g of vitamin D daily, 12 mg of vitamin E daily and 75 $\mu$g of vitamin K daily;

e) design the customised food enriched with at least one nutrient selected from calcium, magnesium, zinc, vitamin A, vitamin B6, vitamin B9, vitamin B12, vitamin C, vitamin D, vitamin E and vitamin K, as well as any of the combinations thereof, based on the results of instructions (c) and (d).

In addition, said computer-implemented system may comprise the additional instruction (f) to store the result of instruction (e) in the memory.

Likewise, said computer-implemented system may further comprise display means configured to display the results of any of the instructions (a) to (e) described above, as well as:

a) a module with a database of the first reference parameter;

b) a module with a database of the second reference parameter;

c) a module with user data corresponding to the individual's genotype at at least one SNP position from among rs17216707 (CYP24A1), rs11144134 (TRPM6), rs1532423 (CA1), rs11645428 (PKD1L2), rs4654748 (NBPF3), rs1801133 (MTHFR), rs601338 (FUT2), rs33972313 (SLC23A1), rs10741657 (CYP2R1), rs12272004 (APOA5) and rs9923231 (VKORC1);

d) a module with user data corresponding to the daily intake of at least one nutrient selected from calcium, magnesium, zinc, vitamin A, vitamin B6, vitamin B9, vitamin B12, vitamin C, vitamin D, vitamin E and vitamin K;

e) a module with a database of nutrients per food; and

f) a module with user preference data.

Another object of the invention is a computer program configured to execute the instructions (a) to (f) defined above.

## DETAILED DESCRIPTION OF THE INVENTION

As described herein, the term "individual" refers to a human of any race, age, height, weight, physical build or level of physical activity.

As described above, a first aspect of the invention relates to a method for designing a customised food for an individual for subsequent manufacture thereof based on said design, wherein the customised food is enriched with at least one nutrient selected from calcium, magnesium, zinc, vitamin A, vitamin B6, vitamin B9, vitamin B12, vitamin C, vitamin D, vitamin E and vitamin K, as well as any of the combinations thereof.

In the context of the invention, the term "calcium" refers to the calcium ion ($Ca^{2+}$). As a nutritional supplement, the calcium ion is preferably supplied as calcium carbonate, calcium citrate, calcium gluconate or calcium lactate. More preferably, the calcium ion is supplied as carbonate and/or citrate.

In the context of the invention, the term "magnesium" refers to the magnesium ion ($Mg^{2+}$). As a nutritional supplement, the magnesium ion is preferably supplied as magnesium oxide, magnesium glycinate, magnesium citrate, magnesium threonate, magnesium chloride and/or magnesium malate.

In the context of the invention, the term "zinc" refers to the zinc ion ($Zn^{2+}$). As a nutritional supplement, the zinc ion is preferably supplied as zinc acetate, zinc aspartate, zinc chloride, zinc citrate, zinc gluconate, zinc oxide and/or zinc sulphate.

In the context of the invention, the term "vitamin A" refers to a group of compounds that includes retinoids such as retinol and its derivatives, retinal and retinoic acid; and several provitamin A carotenoids (especially, beta-carotene).

In the context of the invention, the term "vitamin B6" refers to a group of compounds that includes pyridoxine (or pyridoxol), pyridoxal and pyridoxamine.

In the context of the invention, the term "vitamin B9" refers to the compound also known as vitamin M, folic acid, folacin or pteroylmonoglutamic acid (the anionic form is called folate).

In the context of the invention, the term "vitamin B12" refers to the compound also called cobalamin.

In the context of the invention, the term "vitamin C" refers to the S-enantiomeric compound of ascorbic acid, also called antiscorbutic vitamin.

In the context of the invention, the term "vitamin D" or calciferol refers to two fat-soluble compounds: cholecalciferol (vitamin D3) and ergocalciferol (vitamin D2).

In the context of the invention, the term "vitamin E" refers to several organic compounds made up of several methylated phenols that exist in at least 12 different forms: four tocopherols, four tocomonoenols and four tocotrienols. The tocopherols, tocomonoenols and tocotrienols occur in alpha, beta, gamma, and delta forms, determined by the number and position of methyl groups on the chromanol ring. The four tocopherols are alpha-tocopherol, beta-tocopherol, gamma-tocopherol and delta-tocopherol.

In the context of the invention, the term "vitamin K" refers to a fat-soluble vitamin that comes in two forms: phylloquinone (vitamin K1) and menaquinone (vitamin K2).

In a particular embodiment of the present invention, the method for designing a customised food for an individual, wherein the customised food is enriched with at least one nutrient selected from calcium, magnesium, zinc, vitamin A, vitamin B6, vitamin B9, vitamin B12, vitamin C, vitamin D, vitamin E and vitamin K, includes enriching the customised food according to the following ranges:

- 75-200 mg of calcium per 100 g of customised food, preferably 80-180, 85-160, 90-140, 95-120 or 100-110 mg of calcium per 100 g of customised food, more preferably 75, 80, 85, 90, 95, 100, 110, 120, 130, 140, 150, 160, 170, 180, 190 or 200 mg of calcium per 100 g of customised food;
- 75-120 mg of magnesium per 100 g of customised food, preferably 80-110, 90-100 mg of magnesium per 100 g of customised food, more preferably 75, 90, 100, 110 or 120 mg of magnesium per 100 g of customised food;
- 0.5-3 mg of zinc per 100 g of customised food, preferably 1-2 mg of zinc per 100 g of customised food, more preferably 0.5, 1, 2 or 3 mg of zinc per 100 g of customised food;
- 130-180 $\mu$g of vitamin A per 100 g of customised food, preferably 140-170, 150-160 $\mu$g of vitamin A per 100 g of customised food, more preferably 130, 140, 150, 160, 180 $\mu$g of vitamin A per 100 g of customised food;
- 0.1-0.5 mg of vitamin B6 per 100 g of customised food, preferably 0.2-0.4 mg of vitamin B6 per 100 g of customised food, more preferably 0.1, 0.2, 0.3, 0.4 or 0.5 mg of vitamin B6 per 100 g of customised food;
- 20-80 $\mu$g of vitamin B9 per 100 g of customised food, preferably 30-70, 40-60 $\mu$g of vitamin B9 per 100 g of customised food, more preferably 20, 30, 40, 50, 60, 70 or 80 $\mu$g of vitamin B9 per 100 g of customised food;
- 0.2-0.7 $\mu$g of vitamin B12 per 100 g of customised food, preferably 0.3-0.6, 0.4-0.5 $\mu$g of vitamin B12 per 100 g of customised food, more preferably 0.2, 0.3, 0.4, 0.5, 0.6 or 0.7 $\mu$g of vitamin B12 per 100 g of customised food;
- 10-30 mg of vitamin C per 100 g of customised food, preferably 15-25 mg of vitamin C per 100 g of customised food;
- 0.5-3 $\mu$g of vitamin D per 100 g of customised food, preferably 1-2 $\mu$g of vitamin D per 100 g of customised food;
- 1-8 mg of vitamin E per 100 g of customised food, preferably 2-7, 3-6, 4-5 mg of vitamin E per 100 g of customised food; and/or
- 10-60 $\mu$g of vitamin K per 100 g of customised food, preferably 20-50, 30-40 $\mu$g of vitamin K per 100 g of customised food.

In a particular embodiment of the present invention, the method for preparing a customised food for an individual, wherein the customised food is enriched with at least one nutrient selected from calcium, magnesium, zinc, vitamin A, vitamin B6, vitamin B9, vitamin B12, vitamin C, vitamin D, vitamin E and vitamin K, includes enriching the at least one enriched nutrient in the customised food according to the following ranges:

- 100 mg of calcium per 100 g of customised food;
- 120 mg of magnesium per 100 g of customised food;
- 0.6 mg of zinc per 100 g of customised food;
- 140 $\mu$g of vitamin A per 100 g of customised food;
- 0.3 $\mu$g of vitamin B6 per 100 g of customised food;
- 70 $\mu$g of vitamin B9 per 100 g of customised food;
- 0.6 $\mu$g of vitamin B12 per 100 g of customised food;
- 20 mg of vitamin C per 100 g of customised food;
- 1 $\mu$g of vitamin D per 100 g of customised food;
- 2 mg of vitamin E per 100 g of customised food; and/or
- 40 $\mu$g of vitamin K per 100 g of customised food.

In a particular embodiment of the present invention, the customised food is a customised beverage, preferably wherein the customised beverage is a customised shake.

In a particular embodiment of the invention, in addition to the design of the customised food for an individual, the method will also comprise preparing a health recommendation report for said individual based on a combination of data related to the individual's physical state, together with data related to their eating habits and physical activity and data informing about the genetic identity of said individual in certain SNP positions that offer information about the individual's predispositions to certain nutrient deficiencies, or information about how the individual responds to certain types of diet, physical activity or sleeping habits.

In a first block of steps for preparing the recommendation report, the method will comprise the following sub-steps:

(a) calculating basal metabolic rate (BMR) from data on the individual's age, height and weight;
(b) determining the individual's level of physical activity, where each level of physical activity is assigned a value as an activity factor (AF),
(c) calculating the energy needs in terms of the individual's recommended energy intake (REI), using the formula REI = BMR x AF;
(d) determining the individual's dietary energy intake (DEI), based on data on said individual's food intake frequency; and
(e) recommending that the individual reduce their actual energy intake through changes in diet and/or increased physical activity, if the dietary energy intake (DEI) is greater than the recommended energy intake (REI).

In the context of the present invention, the term "basal metabolic rate" (BMR), "resting metabolic rate" (RMR), or "resting metabolic expenditure" (RME) refers to the rate of energy expenditure per unit of time for an endothermic animal at rest. In the case of the present invention, this refers to a human. BMR is usually expressed in units of energy per unit of time, such as, for example: joule per hour per kg of body mass J/(h·kg) or watts (joule/second).

In an embodiment of the invention, basal metabolic rate (BMR) or resting metabolic expenditure is calculated using the original Harris-Benedict equation:

$BMR = 66.473 + (13.7516 \times$ weight in kg$) + (5.0033 \times$ height in cm$) - (6.755 \times$ age in years$)$ for men, or $BMR = 655.0955 + (9.5634 \times$ weight in kg$) + (1.8496 \times$ height in cm$) - (4.6756 \times$ age in years$)$ for women.

In other embodiments of the invention, resting metabolic expenditure is calculated using any one of the revised versions of the Harris-Benedict equation, which are known to the person skilled in this technical field. In other embodiments of the invention, resting metabolic expenditure is calculated using the Mifflin-St Jeor equation, or using the Katch-McArdle formula.

The term "physical activity" refers to a body movement that requires energy expenditure by an individual. The term "physical activity" thus refers to any movement in any area and includes, without limitation, physical exercise and sport. Activities such as working, walking or cleaning can also be considered a physical activity in the context of the invention. Thus, an "activity factor", also called metabolic constant, can be established, which is associated with the level of physical activity and therefore relates BMR to the energy expenditure required in each category of activity.

In this way, the activity factor varies with each specific activity. Thus, the activity factor is equal to "1" when the level of physical activity is zero, that is, when at rest. In an embodiment of the invention, the level of physical activity to determine the activity factor is selected from light, moderate and high. In another embodiment of the invention, the level of physical activity to determine the activity factor can be selected from rest, very light, light, moderate and intense. In the context of the invention, the person skilled in the art can derive, from the common general knowledge in this technical field, values for the activity factor that are associated with specific activities. In a particular embodiment of the present invention, the activity factor can be established according to table I.

Table I: Activity factor separated by gender, according to level of activity

| Level of activity | Light | Moderate | High |
|---|---|---|---|
| Men | 1.55 | 1.78 | 2.10 |
| Women | 1.56 | 1.64 | 1.82 |

The value of the activity factor (AF) makes it possible to estimate the recommended energy intake (REI), also called recommended energy expenditure, of the individual for activities at a given level of physical activity, using the formula:

$$REI = BMR \times AF.$$

This formula makes it possible to estimate the amount of energy that the individual must obtain through diet to meet the energy needs required to maintain a certain level of activity. That is, the value of recommended energy intake (REI) is related to actual energy needs.

Moreover, in the context of the present invention, dietary energy intake (DEI) is also defined, which corresponds to the value of energy that the individual takes in through diet. This value is also an estimate and it is obtained from a food intake frequency questionnaire. In a food intake frequency questionnaire, the individual indicates what foods they consume after a certain period of time, how often and in what quantity.

Once these two estimated values, recommended energy intake (REI) and dietary energy intake (DEI), are obtained, the following three situations can be established:

- recommended energy intake (REI) is higher than dietary energy intake (DEI), which is indicative of a situation in which the individual loses weight;
- recommended energy intake (REI) is the same as dietary energy intake (DEI), which is indicative of a situation in which the individual's weight remains stable.
- recommended energy intake (REI) is lower than dietary energy intake (DEI), which is indicative of a situation in which the individual gains weight.

In view of the three possible situations, once the recommended energy intake (REI) and dietary energy intake (DEI) values have been estimated, the method of the present invention makes it possible to establish suitable recommendations through changes in food and/or increased physical activity if the individual wishes to lose weight. That is, the method of the invention makes it possible to recommend that the individual reduce their actual energy intake through changes in diet and/or increased physical activity, if the dietary energy intake (DEI) is greater than the recommended energy intake (REI).

In a particular embodiment of the invention, the recommendations report may also include a genetic report of the individual with the results obtained from an additional method that comprises:

i. determining the individual's genotype at the SNP positions rs1042714 (ADRB2), rs1121980 (FTO), rs9939609 (FTO) and rs2272382 (TUB) to estimate the risk of being overweight, where the GG or CG genotype at position rs1042714 (ADRB2), AA or GA genotype at position rs1121980 (FTO), AA or TA genotype at position rs9939609 (FTO) and/or TT or TC genotype at position rs2272382 (TUB) is indicative of a greater predisposition to become overweight;

ii. determining the individual's genotype at the SNP positions rs1042714 (ADRB2), rs1121980 (FTO) and rs1805094 (LEPR) to estimate the risk of gaining weight in case of low frequency of physical activity, where the GG or GC genotype at position rs1042714 (ADRB2), AA or AG genotype at position rs1121980 (FTO) and GG, CC or CG genotype at position rs1805094 (LEPR) is indicative of a greater predisposition to gain weight if the individual has low levels of physical activity;

iii. determining the individual's genotype at the SNP positions rs1801260 (CLOCK) and rs10830963 (MTNR1B) to estimate circadian cycles, where the CC or CT genotype at position rs1801260 (CLOCK) and CC or CG genotype at position rs10830963 (MTNR1B) is indicative of a predisposition to have higher levels of activity during the night;

iv. determining the individual's genotype at the SNP positions rs2943641 (IRS1), rs236918 (PCSK7) and rs12255372 (TCF7L2) to estimate sensitivity to carbohydrate intake, where the CC genotype at position rs2943641 (IRS1); GG genotype at position rs236918 (PCSK7) and the TT or TG genotype at position rs12255372 (TCF7L2) is indicative of greater weight loss on low-calorie diets rich in carbohydrates;

v. determining the individual's genotype at the SNP positions rs1558902 (FTO), rs9939609 (FTO), rs10830963 (MTNR1B) and rs987237 (TFAP2B) to estimate sensitivity to protein intake, where the AA or AT genotype at position rs1558902 (FTO), AA genotype at position rs9939609 (FTO), GG or GC genotype at position rs10830963 (MTNR1B) and GG or GA genotype at position rs987237 (TFAP2B) is indicative of greater weight loss on high-protein diets;

vi. determining the individual's genotype at the SNP positions rs662799 (APOA5), rs2287019 (GIPR), rs1440581 (PPM1K), rs7903146 (TCF7L2) and rs987237 (TFAP2B) to estimate sensitivity to total fat intake, where the CC genotype at position rs662799 (APOA5), TT or TC genotype at position rs2287019 (GIPR), CC or CT genotype at position rs1440581 (PPM1K), TT or TC genotype at position rs7903146 (TCF7L2) and AA genotype at position rs987237 (TFAP2B) is indicative of greater weight loss on low-fat diets and/or greater weight gain on high-fat diets;

vii. determining the individual's genotype at the SNP positions rs9939609 (FTO) and rs1801282 (PPARg2) to estimate sensitivity to saturated and unsaturated fatty acid intake, where the AA or AT genotype at position rs9939609 (FTO) and the GG or GC genotype at position rs1801282 (PPARg2) is indicative of greater weight loss when consuming diets low in saturated fats compared to unsaturated fats and/or greater weight loss in diets enriched in monounsaturated fats;

viii. determining the individual's genotype at the SNP positions rs964184 (ZPR1) and rs2070895 (LIPC) to estimate sensitivity to cholesterol and triglyceride intake, where the GG or GC genotype at position rs964184 (ZPR1) and AA or AG genotype at position rs2070895 (LIPC) is indicative of a better response to low-fat, low-calorie diets;

ix. determining the individual's genotype at the SNP positions rs670 (APOA1), rs2069827 (IL6), rs3812316 (MLXIPL) and rs10830963 (MTNR1B) to estimate the benefit of following a Mediterranean diet, where the AA or AG genotype at position rs670 (APOA1), CC or CG genotype at position rs2069827 (IL6), CC or CG genotype at position rs3812316 (MLXIPL) and CC or CG genotype at position rs10830963 (MTNR1B) is indicative of greater weight loss on a low-calorie Mediterranean diet;

x. determining the individual's genotype at the SNP position rs17216707 (CYP24A1) to estimate the risk of predisposition to low calcium levels, where the TT or TC genotype at position rs17216707 (CYP24A1) is indicative of a risk of calcium deficiency;

xi. determining the individual's genotype at the SNP position rs11144134 (TRPM6) to estimate the risk of predisposition to low magnesium levels, where the AA or AG genotype at position rs11144134 (TRPM6) is indicative of a risk of magnesium deficiency;

xii. determining the individual's genotype at the SNP position rs1532423 (CA1) to estimate the risk of predisposition to low zinc levels, where the TT or TC genotype at position rs1532423 (CA1) is indicative of a greater risk of zinc deficiency;

xiii. determining the individual's genotype at the SNP position rs11645428 (PKD1L2) to estimate the risk of predisposition to low vitamin A levels, the CC genotype at position rs11645428 (PKD1L2) is indicative of a greater risk of vitamin A deficiency;

xiv. determining the individual's genotype at the SNP position rs4654748 (NBPF3) to estimate the risk of predisposition to low vitamin B6 levels, where the CC or CT genotype at position rs4654748 (NBPF3) is indicative of a greater risk of vitamin B6 deficiency;

xv. determining the individual's genotype at the SNP position rs1801133 (MTHFR) to estimate the risk of predisposition to low vitamin B9 levels, where the TT or TC genotype at position rs1801133 (MTHFR) is indicative of a greater risk of vitamin B9 deficiency;

xvi. determining the individual's genotype at the SNP position rs601338 (FUT2) to estimate the risk of predisposition to low vitamin B12 levels, where the GG or GA genotype at position rs601338 (FUT2) is indicative of a greater risk of vitamin B12 deficiency;

xvii. determining the individual's genotype at the SNP position rs33972313 (SLC23A1) to estimate the risk of predisposition to low vitamin C levels, where the AA or AG genotype at position rs33972313 (SLC23A1) is indicative of a greater risk of vitamin C deficiency;

xviii. determining the individual's genotype at the SNP position rs10741657 (CYP2R1) to estimate the risk of predisposition to low vitamin D levels, where the CC or CT genotype at position rs10741657 (CYP2R1) is indicative of a greater risk of vitamin D deficiency;

xix. determining the individual's genotype at the SNP position rs12272004 (APOA5) to estimate the risk of predisposition to low vitamin E levels, where the CC genotype at position rs12272004 (APOA5) is indicative of a greater risk of vitamin E deficiency;

xx. determining the individual's genotype at the SNP position rs9923231 (VKORC1) to estimate the risk of predisposition to low vitamin K levels, where the AA or AG genotype at position rs9923231 (VKORC1) is indicative of a greater risk of vitamin K deficiency;

xxi. determining the individual's genotype at the SNP positions rs5400 (SLC2A2) and rs35874116 (TAS1R2) to estimate food preferences for sweet flavour, where the AA or AG genotype at position rs5400 (SLC2A2) and GG genotype at position rs35874116 (TAS1R2) is indicative of greater perception of sweet flavour;

xxii. determining the individual's genotype at the SNP positions rs713598 (TAS2R38), rs1726866 (TAS2R38) and rs10246939 (TAS2R38) to estimate food preferences for bitter flavour, where the GG or CG genotype at position rs713598 (TAS2R38), CC or CT genotype at position rs1726866 (TAS2R38) and GG or GA genotype at position rs10246939 (TAS2R38) is indicative of greater perception of bitter flavour;

xxiii. determining the individual's genotype at the SNP position rs239345 (SCNN1B) to estimate food preferences for salty flavour, where the AA genotype at position rs239345 (SCNN1B) is indicative of a lower perception of salty flavour;

xxiv. determining the individual's genotype at the SNP position rs1761667 (CD36) to estimate food preferences for fats, where the AA or AG genotype at position rs1761667 (CD36) is indicative of a lower perception of the flavour of fats and oils;

xxv. determining the individual's genotype at the SNP position rs4988235 (MCM6) to estimate the degree of sensitivity to lactose, where the CT genotype at the SNP position rs4988235 (MCM6) is indicative of a risk of having difficulty in digesting lactose in adulthood, while the CC genotype at position rs4988235 (MCM6) is indicative of a risk of lactose intolerance in adulthood;

xxvi. determining the individual's genotype at the SNP position rs762551 (CYP1A2*1F) to estimate the degree of sensitivity to caffeine, where the CA genotype at position rs762551 (CYP1A2*1F) is indicative of intermediate caffeine metabolism, while the CC genotype at position rs762551 (CYP1A2*1F) is indicative of slow caffeine metabolism;

xxvii. determining the individual's genotype at the SNP position rs671 (ALDH2) to estimate the degree of sensitivity to alcohol, where the AA or AG genotype at position rs671 (ALDH2) is indicative of less efficient alcohol metabolism;

xxviii. determining the individual's genotype at the SNP positions rs9939609 (FTO), rs17782313 (MC4R) and rs17066866 (MC4R) to estimate appetite and eating habits, where the AA genotype at position rs9939609 (FTO), CC or CT genotype at position rs17782313 (MC4R) and TT or TA genotype at position rs17066866 (MC4R) is indicative of the risk of snacking between meals; and/or

xxix. determining the individual's genotype at positions rs2395182 (HLA-DRA), rs7775228 (HLA-DQB1), rs4713586 (HLA-DQ4), rs4639334 (HLA-DQ7), rs7454108 (HLA-DQ8) and rs2187668 (HLA-DQA1), to estimate the risk of developing gluten intolerance, where the combination of the T allele at position rs2395182 (HLA-DRA), the G allele at position rs7775228 (HLA-DQB1), and the A, T or G allele at position rs4713586 (HLA-DQ4); the A allele at position rs4639334 (HLA-DQ7); the G allele at position rs7454108 (HLA-DQ8) and the T allele at position rs2187668 (HLA-DQA1) is indicative of the risk of developing gluten intolerance.

As used herein:

- the term "ADRB2" refers to the gene that encodes the beta-2 adrenoceptor protein. The human gene is shown in the Ensembl database under accession number ENSG00000169252;
- the term "ALDH2" refers to the gene that encodes the aldehyde dehydrogenase 2 protein. The human gene is shown in the Ensembl database under accession number ENSG00000111275;
- the term "APOA1" refers to the gene that encodes the apolipoprotein A1 protein. The human gene is shown in the Ensembl database under accession number ENSG00000118137;
- the term "APOA5" refers to the gene that encodes the apolipoprotein A5 protein. The human gene is shown in the Ensembl database under accession number ENSG00000110243;
- the term "CA1" refers to the gene that encodes the carbonic anhydrase 1 protein. The human gene is shown in the Ensembl database under accession number ENSG00000133742;
- the term "CD36" refers to the gene that encodes the CD36 protein. The human gene is shown in the Ensembl database under accession number ENSG00000135218;
- the term "CLOCK" refers to the gene that encodes the circadian regulator clock protein. The human gene is shown in the Ensembl database under accession number ENSG00000134852;
- the term "CYP1A2*1F" refers to the gene that encodes the cytochrome P450 family 1 subfamily A member 2 protein. The human gene is shown in the Ensembl database under accession number ENSG00000140505;
- the term "CYP24A1" refers to the gene that encodes the cytochrome P450 family 24 subfamily A member 1 protein. The human gene is shown in the Ensembl database under accession number ENSG00000019186;
- the term "CYP2R1" refers to the gene that encodes the cytochrome P450 family 2 subfamily R member 1 protein. The human gene is shown in the Ensembl database under accession number ENSG00000186104;
- the term "FTO" refers to the gene that encodes the alpha-ketoglutarate-dependent dioxygenase FTO protein. The human gene is shown in the Ensembl database under accession number ENSG00000140718;
- the term "FUT2" refers to the gene that encodes the fucosyltransferase 2 protein. The human gene is shown in the Ensembl database under accession number ENSG00000176920;
- the term "GIPR" refers to the gene that encodes the gastric inhibitory polypeptide receptor protein. The human gene is shown in the Ensembl database under accession number ENSG00000010310;
- the term "IL6" refers to the gene that encodes the interleukin-6 protein. The human gene is shown in the Ensembl database under accession number ENSG00000136244;
- the term "IRS1" refers to the gene that encodes the insulin receptor substrate 1 protein. The human gene is shown in the Ensembl database under accession number ENSG00000169047;
- the term "LEPR" refers to the gene that encodes the leptin receptor protein. The human gene is shown in the Ensembl database under accession number ENSG00000116678;
- the term "LIPC" refers to the gene that encodes the lipase C protein, hepatic type. The human gene is shown in the Ensembl database under accession number ENSG00000166035;
- the term "MC4R" refers to the gene that encodes the melanocortin 4 receptor protein. The human gene is shown in the Ensembl database under accession number ENSG00000166603;
- the term "MCM6" refers to the gene that encodes the minichromosome maintenance complex component 6 protein. The human gene is shown in the Ensembl database under accession number ENSG00000076003;
- the term "MLXIPL" refers to the gene that encodes the MLX interacting protein-like protein. The human gene is shown in the Ensembl database under accession number ENSG00000009950;
- the term "MTHFR" refers to the gene that encodes the methylenetetrahydrofolate reductase protein. The human gene is shown in the Ensembl database under accession number ENSG00000177000;
- the term "MTNR1B" refers to the gene that encodes the melatonin receptor 1B protein. The human gene is shown in the Ensembl database under accession number ENSG00000134640;
- the term "NBPF3" refers to the gene that encodes the NBPF member 3 protein. The human gene is shown in the Ensembl database under accession number ENSG00000142794;

- the term "PCSK7" refers to the gene that encodes the proprotein convertase subtilisin/kexin type 7 protein. The human gene is shown in the Ensembl database under accession number ENSG00000160613;
- the term "PKD1L2" refers to the gene that encodes the polycystin 1 like 2 protein. The human gene is shown in the Ensembl database under accession number ENSG00000166473;
- the term "PPARG2" refers to the gene that encodes the peroxisome proliferator-activated receptor gamma protein. The human gene is shown in the Ensembl database under accession number ENSG00000132170;
- the term "PPM1K" refers to the gene that encodes the protein phosphatase, Mg2+/Mn2+ dependent 1K protein. The human gene is shown in the Ensembl database under accession number ENSG00000163644;
- the term "SCNN1B" refers to the gene that encodes the sodium channel epithelial 1 beta subunit protein. The human gene is shown in the Ensembl database under accession number ENSG00000168447;
- the term "SLC23A1 " refers to the gene that encodes the solute carrier family 23 member 1 protein. The human gene is shown in the Ensembl database under accession number ENSG00000170482;
- the term "SLC2A2" refers to the gene that encodes the solute carrier family 2 member 2 protein. The human gene is shown in the Ensembl database under accession number ENSG00000163581;
- the term "TAS1R2" refers to the gene that encodes the taste 1 receptor member 2 protein. The human gene is shown in the Ensembl database under accession number ENSG00000179002;
- the term "TAS2R38" refers to the gene that encodes the taste 2 receptor member 38 protein. The human gene is shown in the Ensembl database under accession number ENSG00000257138;
- the term "TCF7L2" refers to the gene that encodes the transcription factor 7 like 2 protein. The human gene is shown in the Ensembl database under accession number ENSG00000148737;
- the term "TFAP2B" refers to the gene that encodes the transcription factor AP-2 beta protein. The human gene is shown in the Ensembl database under accession number ENSG00000008196;
- the term "TRPM6" refers to the gene that encodes the transient receptor potential cation channel subfamily M member 6 protein. The human gene is shown in the Ensembl database under accession number ENSG00000119121;
- the term "TUB" refers to the gene that encodes the TUB bipartite transcription factor protein. The human gene is shown in the Ensembl database under accession number ENSG00000166402;
- the term "VKORC1" refers to the gene that encodes the vitamin K epoxide reductase complex subunit 1 protein. The human gene is shown in the Ensembl database under accession number ENSG00000167397;
- the term "ZPR1" refers to the gene that encodes the ZPR1 zinc finger protein. The human gene is shown in the Ensembl database under accession number ENSG00000109917;
- the term "HLA-DQA1" refers to the gene that encodes the major histocompatibility complex, class II, DQ alpha 1 protein. The human gene is shown in the Ensembl database under accession number ENSG00000196735;
- the term "HLA-DQB1" refers to the gene that encodes the major histocompatibility complex, class II, DQ beta 1 protein. The human gene is shown in the Ensembl database under accession number ENSG00000179344;
- the term "HLA-DRA" refers to the gene that encodes the major histocompatibility complex, class II, DR alpha protein. The human gene is shown in the Ensembl database under accession number ENSG00000204287;
- the term "HLA-DQ" refers to heterodimer conformations of the HLA-DQA1 and HLA-DQB1 proteins.

Genetic information may be provided in the report as bullet points that may consist of semaphores, preferably grouped by categories, for example, regarding nutrition (tendency to gain weight, response to carbohydrate intake, glycaemic index risk, response to fat intake, etc.) or micronutrient metabolism (predisposition to a low level of at least one certain nutrient).

In an embodiment of the present invention, health recommendations are recommendations on diet, physical activity and hours of sleep, such as, for example, following a low-calorie diet (52% carbohydrates, 25% fat and 23% protein with an intake of 1520 kcal/day) or maintaining an exercise routine over a certain number of days per week, in order to contribute to maintaining body weight.

### EXAMPLES

The following invention is described using the following examples, which should be interpreted as merely illustrative and not limiting the scope of the invention.

Example 1: Determination of total energy expenditure

In the context of the present invention, it is necessary to know an individual's total energy expenditure (or total energy intake).

To that end, basic physical data such as gender, age, weight and height are first obtained. With this data, body mass index (BMI) is first calculated, according to the formula:

$$BMI = Weight\ (kg)/[Height\ (m)]^2$$

Based on the BMI result, the following categories can be established:

| BMI | CLASSIFICATION |
|---|---|
| ≤ 18.49 | Underweight |
| 18.50 - 24.99 | Normal weight |
| 25.00 - 26.99 | Class I overweight |
| 27.00 - 29.99 | Class II overweight |
| 30.00 - 34.99 | Type I obesity |
| 35.00 - 39.99 | Type II obesity |
| 40.00 - 49.99 | Type III obesity (morbid) |
| ≥ 50.00 | Type IV obesity (extreme) |

Next, the individual's degree of physical activity is determined, where the individual must answer a questionnaire with questions (and answers) such as those shown below.

1. Given your current weight, do you consider yourself...?

- Obese
- Overweight
- Normal
- Thin
- Do not know/ No answer

2. Compared to one year ago, your current weight is...?

- Higher
- Lower
- The same
- Do not know/ No answer

3. How much weight have you gained/lost in the last year?

- I am the same weight
- I gained between 1 and 3 kg
- I gained more than 3 kg
- I lost between 1 and 3 kg
- I lost more than 3 kg

4. Considering your overall physical activity (main activity, home and free time), what to you consider it to be?

- Light physical activity (People who spend several hours a day in sedentary activities, do not regularly practice sports, use the car to get around, spend most of their leisure time watching TV, reading, using the computer or playing video games. For example, sitting or standing most of the time, walking on flat ground, carrying out light household chores, playing cards, sewing, cooking, studying, driving, typing, office employees, etc.).
- Moderate physical activity 2 or 3 times a week (Walking at 5 km/hour, carrying out tough household chores (cleaning, sweeping, etc.), carpenters, construction workers (except hard labour), chemical industry, electrical industry, mechanised agricultural tasks, golf, childcare, etc. Activities in which objects are moved or handled in a moderate manner. Involving more than 30 minutes/day of moderate activity and more than 20 minutes/week of vigorous activity).

- High physical activity (People who walk long distances daily, use the bicycle to get around, carry out vigorous activities or play sports that require a high level of effort for several hours. For example: Non-mechanised agricultural, mining and forestry tasks, digging, chopping firewood, reaping by hand, climbing, mountaineering, playing football, playing tennis, jogging, dancing, skiing, etc. Involving moderate or vigorous activity every day).

With this data, resting metabolic expenditure, or basal metabolic rate (BMR), is calculated, using the original Harris-Benedict equation:

- BMR = 66.473 + (13.7516 $\times$ weight in kg) + (5.0033 $\times$ height in cm) - (6.755 $\times$ age in years) for men, or
- BMR = 655.0955 + (9.5634 $\times$ weight in kg) + (1.8496 $\times$ height in cm) - (4.6756 $\times$ age in years) for women.

**Total energy expenditure**

Total energy expenditure is calculated by multiplying the resting metabolic rate or basal metabolic rate (BMR) by the physical activity coefficients in this table, according to the type of activity referred to in the questionnaire.

| Activity factor (AF) | Light (1) | Moderate (2) | High (3) |
|---|---|---|---|
| Men | 1.55 | 1.78 | 2.10 |
| Women | 1.56 | 1.64 | 1.82 |

Thus, the energy needs can be expressed in terms of the individual's recommended energy intake (REI) according to the following formula:

$$REI = BMR \times AF$$

Example 2: Determination of eating habits

Eating habits can be determined based on an eating habits questionnaire.

| QUESTION | VALUE |
|---|---|
| *1. How many meals do you eat a day?* | |
| 5 meals a day: 3 main meals, one mid-morning snack and one mid-afternoon snack. | 2 |
| 2-3 large meals. | 0 |
| It depends on the day. | 1 |
| *2. How much time do you spend having lunch or dinner?* | |
| At least 1 hour. | 2 |
| Less than 1/2 hour. | 0 |
| Between 1/2 hour - 1 hour. | 1 |
| *3. What do you usually eat between meals?* | |
| Fruit or vegetables. | 2 |
| Bars, biscuits, crackers. | 0 |
| Yoghurt or nuts. | 1 |
| *4. How often do you think you overeat?* | |
| Less than once a month. | 2 |
| More than once a month. | 0 |
| Once a month. | 1 |
| *5. How many litres of water do you drink a day?* | |

(continued)

| QUESTION | VALUE |
|---|---|
| 2 litres. | 2 |
| I drink little water daily. | 0 |
| At least 1 litre. | 1 |
| 6. What factor do you consider most important when choosing a food to eat? | |
| The nutritional content. | 2 |
| Price. | 0 |
| Flavour. | 1 |
| 7. Is there any food you choose not to eat and why? | |
| To take care of myself. | 2 |
| Because I do not like it. | 0 |
| I usually do not avoid any food. | 1 |
| 8. What is the most common way you prepare your food? | |
| Roasted or grilled. | 2 |
| Fried or stewed. | 0 |
| Steamed or boiled. | 1 |
| 9. What do you do with visible fat on meat? | |
| I remove it all. | 2 |
| I eat it. | 0 |
| I remove most of it. | 1 |
| 10. How much do you smoke? | |
| I do not smoke. | 2 |
| Several cigarettes a day. | 0 |
| Occasionally. | 1 |
| 11. How often do you eat in restaurants? | |
| Once a week or less. | 2 |
| I usually go very frequently. | 0 |
| I usually go at least 2 times a week. | 1 |
| 12. Which of the following answers reflects your alcohol consumption? | |
| I do not drink, but I occasionally have a glass of wine. | 2 |
| I drink something every day. | 0 |
| Only on weekends. | 1 |
| 13. How many cups of coffee do you drink a day? | |
| No more than 2 cups a day. | 2 |
| More than 4 cups a day. | 0 |
| Between 2-4 cups a day. | 1 |
| 14. What do you do when you feel satiated? | |
| I stopped eating without a problem. | 2 |
| I continue eating. | 0 |
| I stop eating, but it's hard for me to do. | 1 |

(continued)

| QUESTION | VALUE |
|---|---|
| *15. Do you eat on impulse due to gluttony, anxiety or stress?* | |
| No, I usually eat slowly, trying to chew properly. | 2 |
| Yes, I cannot control those situations. | 0 |
| Occasionally. | 1 |
| *16. How much physical activity do you do?* | |
| About 60 minutes a day or more. | 2 |
| Less than 30 minutes. | 0 |
| At least 30 minutes a day. | 1 |
| *17. What do you do or would be willing to do to take care of your body?* | |
| Watch my diet and exercise. | 2 |
| Nothing. | 0 |
| Follow a temporary diet. | 1 |
| *18. You consider your diet to be...:* | |
| Different every day. | 2 |
| Monotonous. | 0 |
| Different only on weekends. | 1 |

The result of this section is obtained by calculating the sum of the score based on the points obtained in each answer. Thus, the possible result depends on the score obtained:

- Healthy eating habits: >30 points
- Needs improvement: 20-30
- Bad habits: <20 points

Example 3: Determination of food frequency (FFQ)

To collect more information about the user's lifestyle, a questionnaire has been developed, adapted and validated and it asks questions about the daily consumption of different types of foods, grouped by categories, and the quantities of their consumption (the reference units are grams in the case of solids and millilitres in the case of liquids). The food frequency questionnaire (FFQ) is shown below.

| FOOD FREQUENCY | | | |
|---|---|---|---|
| *I. DAIRY* | | | |
| 1. Whole milk | 160 | 250 | 325 |
| 2. Semi-skimmed milk | 160 | 250 | 325 |
| 3. Non-fat or skimmed milk | 160 | 250 | 325 |
| 4. Whole yoghurt | 80 | 125 | 140 |
| 5. Non-fat yoghurt | 80 | 125 | 140 |
| 6. Curd, white or fresh cheese | 75 | 150 | 300 |
| 7. Aged, semi-aged or cream cheese | 20 | 50 | 80 |
| 8. Custard, flan, pudding | 75 | 150 | 300 |
| 9. Ice cream | 75 | 100 | 150 |

(continued)

| II. EGGS, MEAT, FISH | | | |
|---|---|---|---|
| 10. Chicken eggs | 65 | 130 | 195 |
| 11. Chicken with skin | | 150 | 250 |
| 12. Skinless chicken | | 150 | 250 |
| 13. Veal, pork, lamb (as a main dish) | 80 | 175 | 350 |
| 14. Beef, pork, chicken liver | 80 | 175 | 350 |
| 15. Cured meat: ham, pepperoni, salami | 25 | 50 | 75 |
| 16. Sausages and the like | 40 | 80 | 160 |
| 17. Pates, foie gras | 25 | 50 | 100 |
| 18. Hamburgers | 80 | 130 | 200 |
| 19. Assorted fried fish | 50 | 100 | 150 |
| 20. Boiled or grilled white fish: hake, sole | | 100 | 200 |
| 21. Boiled or grilled oily fish: sardines, tuna | | 150 | 200 |
| 22. Canned fish: tuna, sardines, herrings | 40 | 80 | 120 |
| 23. Clams, mussels, oysters | 100 | 200 | 400 |
| 24. Squid, baby squid, cuttlefish, octopus | 80 | 130 | 200 |
| 25. Seafood: shrimp, crab, prawn, lobster | 50 | 100 | 150 |
| III. VEGETABLES, LEGUMES, FRUIT | | | |
| 26. Cooked spinach or chard | 100 | 200 | 300 |
| 27. Cooked cabbage, cauliflower, broccoli | 100 | 175 | 300 |
| 28. Lettuce, endives, escarole | 40 | 80 | 120 |
| 29. Tomato | 90 | 150 | 250 |
| 30. Onion | 25 | 50 | 100 |
| 31. Carrot, pumpkin | 50 | 100 | 150 |
| 32. Cooked green beans | 100 | 200 | 300 |
| 33. Aubergines, courgettes, cucumbers | 100 | 175 | 250 |
| 34. Peppers | 100 | 180 | 260 |
| 35. Asparagus | 100 | 175 | 250 |
| 36. Artichokes | | 100 | 200 |
| 37. Boiled corn | 50 | 100 | 150 |
| 38. Legumes: lentils, chickpeas, pinto or white beans | 100 | 200 | 300 |
| 39. Oranges, tangerines | | 110 | 190 |
| 40. Fresh orange juice | 150 | 250 | 350 |
| 41. Banana | 110 | 150 | 190 |
| 42. Apple, pear | 110 | 220 | 300 |
| 43. Grapes, strawberries, cherries | 100 | 200 | 300 |
| 44. Peach, nectarine, apricot | | 40 | 120 |
| 45. Watermelon, melon | 150 | 250 | 350 |
| 46. Fruit in syrup | 40 | 80 | 120 |
| 47. Kiwi | | 75 | 150 |

(continued)

| III. VEGETABLES, LEGUMES, FRUIT | | | |
|---|---|---|---|
| 48. Olives | | 20 | 60 |
| 49. Nuts: almonds, peanuts, pine nuts, hazelnuts | 20 | 40 | 60 |
| IV. BREAD, CEREALS AND THE LIKE | | | |
| 50. White bread | 50 | 125 | 250 |
| 51. Wholegrain bread | 50 | 100 | 150 |
| 52. Cooked, roasted potatoes | 100 | 200 | 300 |
| 53. Fried potatoes | 100 | 200 | 300 |
| 54. Potato crisps | 50 | 100 | 125 |
| 55. Cooked rice | 100 | 200 | 300 |
| 56. Pasta: spaghetti, noodles, macaroni and the like | 100 | 200 | 300 |
| 57. Pizza | 100 | 200 | 400 |
| V. OILS, FATS AND THE LIKE | | | |
| 58. Olive oil | 10 | 20 | 30 |
| 59. Sunflower, corn, soybean oil | 10 | 20 | 30 |
| 60. Margarine added to bread or food | 25 | 50 | 75 |
| 61. Butter added to bread or food | 25 | 50 | 75 |
| 62. Marie-type biscuits | 20 | 40 | 55 |
| 63. Chocolate cookies (filled, with chocolate chips, with raisins) | 15 | 45 | 90 |
| 64. Pastries: croissant, doughnut, cupcake, sponge cake, cake or the like | 80 | 160 | 240 |
| 65. Chocolate, bonbons and the like | | | |
| 66. Chocolate powder, Cola-Cao chocolate powder and the like | | | |
| VI. PRE-COOKED AND MISCELLANEOUS BEVERAGES | | | |
| 67. Red wine | 90 | 180 | 270 |
| 68. White or rosé wine | 90 | 180 | 270 |
| 69. Beer | 200 | 330 | 400 |
| 70. Brandy, gin, rum, whisky, vodka, 40° spirits | | 50 | 100 |
| 71. Sugar-sweetened cola, orange, lemon soft drinks | 200 | 330 | 500 |
| 72. "Light" sugar-free cola, orange, lemon soft drinks | 200 | 330 | 500 |
| 73. Packaged fruit juice | 150 | 250 | 350 |
| 74. Coffee | 50 | 150 | 200 |
| 75. Croquettes | 60 | 100 | 140 |
| 76. Fish sticks, fried fish delicacies and the like | 50 | 100 | 150 |
| 77. Mayonnaise | 15 | 25 | |
| 78. Tomato sauce | 25 | 50 | 75 |
| 79. Ketchup | 15 | 25 | 50 |
| 80. Salt | 3 | 6 | 17 |
| 81. Garlic | 2 | 25 | 50 |
| 83. Jams, honey | | 15 | 25 |
| 83. Sugar (for example, in coffee, desserts, etc.) | 5 | 15 | 25 |

The answers to this questionnaire are integrated with a compositional (nutritional) table of the different foods in order to have an estimate of the quantities consumed by each user over a one-year period, in addition to determining the individual's daily intake of at least one nutrient selected from calcium, magnesium, zinc, vitamin A, vitamin B6, vitamin B9, vitamin B12, vitamin C, vitamin D, vitamin E and vitamin K, as well as any of the combinations thereof, data that is used to design the customised food using the method object of the invention. Using this methodology, the average intake of calories and nutrients (micronutrients and macronutrients) is obtained to establish the recommendations in each section.

The final objective of this activity is to interrelate the habits described by users, where possible deficiencies in the intake of certain nutrients can be observed, with the possible deficiencies determined in the genetic test in order to generate dietary recommendations. To that end, an encrypted database is created to maintain confidentiality at all times.

**Claims**

1. A method for designing a customised food for an individual for subsequent manufacture thereof, wherein the customised food is enriched with at least one nutrient selected from a group consisting of calcium, magnesium, zinc, vitamin A, vitamin B6, vitamin B9, vitamin B12, vitamin C, vitamin D, vitamin E and vitamin K, as well as any of the combinations thereof and wherein the method comprises:

   (a) determining the individual's genotype at at least one SNP position selected from a group consisting of rs17216707, rs11144134, rs1532423, rs11645428, rs4654748, rs1801133, rs601338, rs33972313, rs10741657, rs12272004 and rs9923231, as well as any of the combinations thereof, from a DNA sample of said individual;
   (b) calculating the individual's daily intake of at least one nutrient selected from a group consisting of calcium, magnesium, zinc, vitamin A, vitamin B6, vitamin B9, vitamin B12, vitamin C, vitamin D, vitamin E and vitamin K, as well as any of the combinations thereof;

   where:

   - if the genotype for rs17216707 is TT or TC and/or if the daily calcium intake is less than 800 mg daily, the customised food is enriched in calcium;
   - if the genotype for rs11144134 is AA or AG and/or if the daily magnesium intake is less than 375 mg daily, the customised food is enriched in magnesium;
   - if the genotype for rs1532423 is TT or TC and/or if the daily zinc intake is less than 10 mg daily, the customised food is enriched in zinc;
   - if the genotype for rs11645428 is CC and/or if the daily vitamin A intake is less than 800 $\mu$g daily, the customised food is enriched in vitamin A;
   - if the genotype for rs4654748 is CC or CT and/or if the daily vitamin B6 intake is less than 1.4 mg daily, the customised food is enriched in vitamin B6;
   - if the genotype for rs1801133 is TT or TC and/or if the daily vitamin B9 intake is less than 200 $\mu$g daily, the customised food is enriched in vitamin B9;
   - if the genotype for rs601338 is GG or GA and/or if the daily vitamin B12 intake is less than 2.5 $\mu$g daily, the customised food is enriched in vitamin B12;
   - if the genotype for rs33972313 is AA or AG and/or if the daily vitamin C intake is less than 80 mg daily, the customised food is enriched in vitamin C;
   - if the genotype for rs10741657 is CC or CT and/or if the daily vitamin D intake is less than 5 $\mu$g daily, the customised food is enriched in vitamin D;
   - if the genotype for rs12272004 is CC and/or if the daily vitamin E intake is less than 12 mg daily, the customised food is enriched in vitamin E;
   - and/or if the genotype for rs9923231 is AA or AG and/or if the daily vitamin K intake is less than 75 $\mu$g daily, the customised food is enriched in vitamin K.

2. The method according to claim 1, wherein the food is a beverage.

3. The method according to claim 1 or 2, wherein the calculation of the individual's daily intake of at least one nutrient selected from a group consisting of calcium, magnesium, zinc, vitamin A, vitamin B6, vitamin B9, vitamin B12, vitamin C, vitamin D, vitamin E and vitamin K, as well as any of the combinations thereof, is carried out based on the individual's consumption of at least one food selected from a group consisting of dairy, eggs, meat, fish, vegetables, legumes, fruit, bread, cereals, oils, fats and beverages, wherein each food is defined by the amount of each nutrient selected from the group consisting of calcium, magnesium, zinc, vitamin A, vitamin B6, vitamin B9, vitamin B12, vitamin C, vitamin D, vitamin E and vitamin K, as well as any of the combinations thereof, that is present in it.

4. The method according to any one of the preceding claims, wherein said method additionally comprises determining user preferences that are part of the design of the customised food, wherein said user preferences are selected from a group consisting of a base ingredient of the food, base ingredient being understood as the ingredient that is found in the highest percentage in the food, a flavour of the food, an increase in the percentage of at least one nutrient selected from protein and fibre and a decrease in the percentage of cholesterol, wherein each recommendation is associated with an ingredient, resulting in a formula that defines the nutritional and ingredient composition of said customised food for subsequent manufacture thereof.

5. The method according to claim 4, wherein said method comprises an additional step of storing said formula in a QR code.

6. The method according to claim 4, wherein said method comprises an additional step of storing said formula on a label that is subsequently printed.

7. The method according to claim 6, wherein said label additionally includes data on the amount of total food to be manufactured.

8. The method according to any one of the preceding claims, wherein said method additionally comprises a step of generating a recommendations report on the individual's energy intake that comprises:

(a) calculating the individual's basal metabolic rate (BMR) from data on the individual's age, height and weight;
(b) determining the individual's level of physical activity, where each level of physical activity is assigned a value as an activity factor (AF),
(c) calculating the energy needs in terms of the individual's recommended energy intake (REI), using the formula REI = BMR x AF;
(d) determining the individual's dietary energy intake (DEI), based on data on said individual's food intake frequency; and
(e) recommending that the individual reduce their actual energy intake through changes in diet and/or increased physical activity, if the dietary energy intake (DEI) is greater than the recommended energy intake (REI).

9. A computer-implemented system configured to design a customised food for subsequent manufacture thereof, wherein said food is enriched with at least one nutrient selected from a group consisting of calcium, magnesium, zinc, vitamin A, vitamin B6, vitamin B9, vitamin B12, vitamin C, vitamin D, vitamin E and vitamin K, as well as any of the combinations thereof, wherein said system comprises a processing unit comprising a memory and wherein said processing unit is configured to execute the following instructions:

a) in the memory, store user data corresponding to the individual's genotype at at least one SNP position selected from a group consisting of rs17216707, rs11144134, rs1532423, rs11645428, rs4654748, rs1801133, rs601338, rs33972313, rs10741657, rs12272004 and rs9923231, as well as any of the combinations thereof;
b) in the memory, store user data corresponding to the daily intake of at least one nutrient selected from calcium, magnesium, zinc, vitamin A, vitamin B6, vitamin B9, vitamin B12, vitamin C, vitamin D, vitamin E and vitamin K, as well as any of the combinations thereof;
c) compare said genotype of the individual with a first reference parameter, wherein said first reference parameter refers to a genotype at an SNP position consisting of TT or TC for rs17216707, AA or AG for rs11144134, TT or TC for rs1532423, CC for rs11645428, CC or CT for rs4654748, TT or TC for rs1801133, GG or GA for rs601338, AA or AG for rs33972313, CC or CT for rs10741657, CC for rs12272004 and AA or AG for rs9923231;
d) compare said daily intake of the individual with a second reference parameter, wherein said second reference parameter refers to a recommended daily intake of at least one nutrient consisting of 800 mg of calcium daily, 375 mg of magnesium daily, 10 mg of zinc daily, 800 $\mu$g of vitamin A daily, 1.4 mg of vitamin B6 daily, 200 $\mu$g of vitamin B9 daily, 2.5 $\mu$g of vitamin B12 daily, 80 mg of vitamin C daily, 5 $\mu$g of vitamin D daily, 12 mg of vitamin E daily and 75 $\mu$g of vitamin K daily;
e) design the customised food enriched with at least one nutrient selected from calcium, magnesium, zinc, vitamin A, vitamin B6, vitamin B9, vitamin B12, vitamin C, vitamin D, vitamin E and vitamin K, as well as any of the combinations thereof, based on the results of instructions (c) and (d).

10. The system according to claim 9, wherein said system comprises an additional instruction (f) to store the result of instruction (e) in the memory.

11. The system according to claim 10, wherein said system further comprises display means configured to display the results of any of the instructions (a) to (e), as well as at least one module selected from a group consisting of:

g) a module with a database of the first reference parameter;
h) a module with a database of the second reference parameter;
i) a module with user data corresponding to the individual's genotype at at least one SNP position of a group consisting of rs17216707, rs11144134, rs1532423, rs11645428, rs4654748, rs1801133, rs601338, rs33972313, rs10741657, rs12272004 and rs9923231;
j) a module with user data corresponding to the daily intake of at least one nutrient selected from calcium, magnesium, zinc, vitamin A, vitamin B6, vitamin B9, vitamin B12, vitamin C, vitamin D, vitamin E and vitamin K;
k) a module with a database of nutrients per food; and
l) a module with user preference data.

12. A computer program configured to execute the instructions (a) to (f) of the system according to claim 10.

# EP 4 702 849 A1

## INFORME DE BÚSQUEDA INTERNACIONAL

| | Solicitud internacional Nº |
|---|---|
| | **PCT/ES2024/070173** |

### A. CLASIFICACIÓN DEL OBJETO DE LA SOLICITUD

*A23L 33/15*(2016.01)i; *A23L 33/16*(2016.01)i; *C12Q 1/6883*(2018.01)i; *G01N 33/50*(2006.01)i; *G06Q 30/0601*(2023.01)i; *G16H 10/00*(2018.01)i; *G16H 20/60*(2018.01)i

De acuerdo con la Clasificación Internacional de Patentes (CIP) o según la clasificación nacional y CIP.

### B. SECTORES COMPRENDIDOS POR LA BÚSQUEDA

Documentación mínima buscada (sistema de clasificación seguido de los símbolos de clasificación)

    A23L; G16H; G06Q; G01N; C12Q

Otra documentación consultada, además de la documentación mínima, en la medida en que tales documentos formen parte de los sectores comprendidos por la búsqueda

Bases de datos electrónicas consultadas durante la búsqueda internacional (nombre de la base de datos y, si es posible, términos de búsqueda utilizados)

    EPO-Internal

### C. DOCUMENTOS CONSIDERADOS RELEVANTES

| Categoría* | Documentos citados, con indicación, si procede, de las partes relevantes | Relevante para las reivindicaciones nº |
|---|---|---|
| X | US 2012277180 A1 (MARINI NICHOLAS [US] ET AL) 01 noviembre 2012 (2012-11-01) | 1-7, 9-12 |
| Y | párrafos [0009], [0037], [0048], [0051], [0060], [0061], [0066], [0069] - [0070], [0133] - [0187], [0190] - [0219], [0230] - [0235] | 8 |
| | párrafos [0278] - [0300]; reivindicaciones 1-57 | |
| Y | US 2019099124 A1 (MATTIS JAMES ALEXANDER [US] ET AL) 04 abril 2019 (2019-04-04) | 8 |
| | párrafos [0015], [0052]; reivindicaciones 1-20 | |
| A | CA 3114963 A1 (GINI HEALTH INC [CA]) 28 junio 2021 (2021-06-28) | 1-12 |
| | todo el documento | |
| A | WO 2010030600 A2 (CODE NUTRITION LLC [US]; BEARSS DAVID [US] ET AL.) 18 marzo 2010 (2010-03-18) | 1-12 |
| | todo el documento | |
| A | WO 2015004266 A1 (NOVOGENIA GMBH [AT]) 15 enero 2015 (2015-01-15) | 1-12 |
| | todo el documento | |

☐ En la continuación del Recuadro C se relacionan otros documentos    ☑ Los documentos de familias de patentes se indican en el Anexo

| | |
|---|---|
| \* Categorías especiales de documentos citados: | "T" documento ulterior publicado con posterioridad a la fecha de presentación internacional o de prioridad que no pertenece al estado de la técnica pertinente pero que se cita por permitir la comprensión del principio o teoría que constituye la base de la invención. |
| "A" documento que define el estado general de la técnica no considerado como particularmente relevante. | |
| "E" solicitud de patente o patente anterior pero publicada en la fecha de presentación internacional o en fecha posterior. | "X" documento particularmente relevante: la invención reivindicada no puede considerarse nueva o que implique una actividad inventiva por referencia al documento aisladamente considerado. |
| "L" documento que puede plantear dudas sobre una reivindicación de prioridad o que se cita para determinar la fecha de publicación de otra cita o por una razón especial (como la indicada). | |
| "O" documento que se refiere a una divulgación oral, a una utilización, a una exposición o a cualquier otro medio. | "Y" documento particularmente relevante: la invención reivindicada no puede considerarse que implique una actividad inventiva cuando el documento se asocia a otro u otros documentos de la misma naturaleza, cuya combinación resulta evidente para un experto en la materia. |
| "P" documento publicado antes de la fecha de presentación internacional pero con posterioridad a la fecha de prioridad reivindicada. | "&" documento que forma parte de la misma familia de patentes. |

| Fecha en que se ha concluido efectivamente la búsqueda internacional | Fecha de expedición del informe de búsqueda internacional |
|---|---|
| **07 agosto 2024** | **13 septiembre 2024** |

| Nombre y dirección postal de la Administración encargada de la Funcionario autorizado búsqueda internacional | Funcionario autorizado |
|---|---|
| **European Patent Office** **p.b. 5818, Patentlaan 2, 2280 HV Rijswijk** **Países Bajos (Reino de los)** Nº de teléfono: **(+31-70)340-2040** Nº de fax: **(+31-70)340-3016** | **Schlegel, Birgit** Nº de teléfono: |

Formulario PCT/ISA/210 (segunda hoja) (Enero 2015)

**EP 4 702 849 A1**

| INFORME DE BÚSQUEDA INTERNACIONAL | Solicitud internacional Nº |
|---|---|
| Información relativa a miembros de familias de patentes | **PCT/ES2024/070173** |

| Documento de patente citado en al informe de búsqueda | | | Fecha de publicación (día/mes/año) | Miembro(s) de la familia de patentes | | | Fecha de publicación (día/mes/año) |
|---|---|---|---|---|---|---|---|
| US | 2012277180 | A1 | 01 noviembre 2012 | AU | 2010300475 | A1 | 03 mayo 2012 |
| | | | | BR | 112012007345 | A2 | 01 septiembre 2015 |
| | | | | CA | 2776173 | A1 | 07 abril 2011 |
| | | | | CN | 102781259 | A | 14 noviembre 2012 |
| | | | | EP | 2482677 | A1 | 08 agosto 2012 |
| | | | | JP | 2013506689 | A | 28 febrero 2013 |
| | | | | KR | 20120100964 | A | 12 septiembre 2012 |
| | | | | RU | 2012117898 | A | 10 noviembre 2013 |
| | | | | US | 2012277180 | A1 | 01 noviembre 2012 |
| | | | | WO | 2011041611 | A1 | 07 abril 2011 |
| US | 2019099124 | A1 | 04 abril 2019 | NINGUNO | | | |
| CA | 3114963 | A1 | 28 junio 2021 | NINGUNO | | | |
| WO | 2010030600 | A2 | 18 marzo 2010 | NINGUNO | | | |
| WO | 2015004266 | A1 | 15 enero 2015 | NINGUNO | | | |

Formulario PCT/ISA/210 (anexo_familia de patentes) (Enero 2015)